# EUROPEAN PATENT SPECIFICATION

(11) **EP 2 589 693 B1**
(45) Date of publication and mention of the grant of the patent: **04.10.2017**
(21) Application number: 11800853.1
(22) Date of filing: 28.06.2011
(51) Int. Cl.: D04H 1/72, A61K 8/02, A61K 8/06, A61K 8/73, A61Q 19/00, D01D 5/04, D01D 5/34, D01F 6/00

(54) **NANOFIBER**
NANOFASER
NANOFIBRE

(30) Priority: 29.06.2010 JP 2010148076
(43) Date of publication of application: 08.05.2013
(73) Proprietor: Kao Corporation, Chuo-Ku Tokyo 103-8210 (JP)
(72) Inventor: TOJO, Takehiko, Haga-gun Tochigi 321-3497 (JP); YAMASHITA, Yoshimi, Haga-gun Tochigi 321-3497 (JP); ISHIKAWA, Masataka, Haga-gun Tochigi 321-3497 (JP); SHUIN, Mika, Tokyo 131-0044 (JP); UNO, Shinnosuke, Tokyo 131-0044 (JP)
(74) Representative: Vossius & Partner Patentanwälte Rechtsanwälte mbB
(86) International application number: PCT/JP2011/064809
(87) International publication number: WO 2012/002389

(56) References cited:
- WO-A1-2009/031620
- WO-A1-2010/074212
- WO-A2-2008/015573
- WO-A2-2008/146169
- WO-A2-2009/133059
- JP-A- 2007 197 179
- JP-A- 2008 531 860
- JP-A- 2009 545 307
- JP-A- 2010 229 560
- US-A1- 2006 264 130

## Description

### Technical Field

The present invention relates to a nanofiber and a method for producing the nanofiber. It also relates to a nanofiber sheet.

### Background Art

Techniques relating to water-soluble polymer-based nanofibers are known as disclosed, e.g., in patent literature 1 below. The nanofiber disclosed is prepared by electrospinning a solution of a water soluble polymer in a solvent such as water. The solution is described as optionally containing a functional component, such as an emulsifier, a stabilizer, a bactericide, a moisturizer, and so on. When the solution contains such a functional component, the resulting nanofiber contains the functional component therein.

With regard to incorporation of a functional component into a nanofiber, the technique of patent literature 2 below is also known. The literature discusses a network structure formed of polymer nanofibers holding a cosmetic or a cosmetic ingredient. The cosmetic or the like is held inside the nanofiber. The nanofiber holding the cosmetic or the like is obtained by mixing the cosmetic or the like into a solution of a polymer constructing the nanofiber.

### Citation List

### Patent Literature

Patent literature 1: WO2009/031620
Patent literature 2: JP 2008-179629A
WO2008/146169 A2 discloses a smoking article comprising electroprocessed microcapsules and nanocapsules.
US2006/0264130 A1 discloses fibrous structures comprising electrostatically prepared polysaccharide micro- and nanofibers.
WO2008/015573 A2 discloses a smoking article comprising electrospun microfibers and nanofibers.

### Summary of Invention

The nanofibers described in the patent literatures cited above are likely to feel sticky because the functional component, cosmetic, etc. exists not only inside but on the surface thereof There is a limit to the amount of a functional component or the like that can be incorporated into nanofibers. The functional component existing on the fiber surface tends to suffer from deterioration or denaturation, so that the nanofibers are expected to have poor storage stability.

### Solution to Problem

The invention provides a nanofiber formed of a water soluble polymer, having a cavity, and containing an oily component in the cavity, the nanofiber having a large-diametered portion and a small-diametered portion, and the large-diametered portion having the cavity wherein the oily component contains a solvent selected from squalane, olive oil, silicone oil, macadamia nut oil, or cetyl 1,3-dimethylbutyl ether, and an oil-soluble component selected from vitamin E, chamomile extract, or rose extract, as dissolved in the solvent as an active ingredient.

The invention also provides a suitable method for producing the nanofibers of the invention. The method includes electrospinning an O/W emulsion having a water soluble polymer dissolved in the aqueous phase and an oily component contained in an oily phase. The invention also provides a method for keeping a surface of an object moistured,

### as stated in claim 14.

The present application discloses another suitable method for producing the nanofiber. The method includes providing a first liquid having a water soluble polymer dissolved in water and a second liquid containing an oily component and electrospinning the first liquid and the second liquid using a duplex-tube capillary having a sheath and a core. The first liquid is fed to the sheath, and the second liquid is fed to the core.

### The present application discloses the following subject matter.

[1] A nanofiber made from a water soluble polymer, having a cavity, and containing an oily component in the cavity.
[2] The nanofiber set forth in [1] above, wherein the oily component in the cavity is liquid at ambient temperature.
[3] The nanofiber set forth in [1] or [2] above, having a large-diametered portion and a small-diametered portion, the large-diametered portion having the cavity.
[4] The nanofiber set forth in [1] or [2] above, having a large-diametered portion and a small-diametered portion, both the large-diametered portion and the small-diametered portion having the cavity, and the cavity in the large-diametered portion and the cavity in the small-diametered portion being interconnected.
[5] The nanofiber set forth in [1] or [2] above, having a tubular shape with a substantially constant outer diameter and a substantially constant inner diameter.
[6] The nanofiber set forth in any one of [1] to [5], wherein the water soluble polymer is a naturally occurring polymer, such as a mucopolysaccharide, e.g., pullulan, hyaluronic acid, chondroitin sulfate, poly-γ-glutamic acid, modified corn starch, β-glucan, gluco-oligosaccharide, heparin, and keratosulfate, cellulose, pectin, xylan, lignin, glucomannan, galacturonic acid, psyllium seed gum, tamarind seed gum, gum arabic, tragacanth gum, soybean water-soluble polysaccharide, alginic acid, carrageenan, laminaran, agar (agarose), fucoidan, methyl cellulose, hydroxypropyl cellulose, and hydroxypropylmethyl cellulose; or a synthetic polymer, such as partially saponified polyvinyl alcohol, low-saponified polyvinyl alcohol, polyvinylpyrrolidone, polyethylene oxide, and sodium polyacrylate.
[7] The nanofiber set forth in any one of [1] to [5], wherein the water soluble polymer is pullulan or a synthetic polymer selected from the group consisting of partially saponified polyvinyl alcohol, low-saponified polyvinyl alcohol, polyvinylpyrrolidone, and polyethylene oxide.
[8] The nanofiber set forth in [6] or [7], wherein the water soluble polymers are used individually or in combination of two or more thereof.
[9] The nanofiber set forth in any one of [1] to [5], wherein the water soluble polymer is pullulan.
[10] The nanofiber set forth in any one of [1] to [9], wherein the oily component contains a solvent selected from squalane, olive oil, silicone oil, macadamia nut oil, and cetyl 1,3-dimethylbutyl ether.
[11] The nanofiber set forth in [10], wherein the oily component further contains an oil-soluble component selected from vitamin E, chamomile extract, and rose extract as dissolved in the solvent as an effective ingredient.
[12] The nanofiber set forth in [10] or [11], wherein the oily components are used either individually or in combination of two or more thereof.
[13] The nanofiber set forth in any one of [1] to [12], wherein the oily component comprising a first oily component and a second oily component, the cavity comprising a plurality of cavities discretely formed over the whole length of the nanofiber, and each cavity contains both the first oily component and the second oily component.
[14] The nanofiber set forth in any one of [1] to [12], wherein the oily component contains a first oily component and a second oily component, the cavity comprising a plurality of cavities discretely formed over the whole length of the nanofiber, some of the cavities contain the first oily component and do not contain the second oily component, and others contain the second oily component and do not contain the first oily component.
[15] The nanofiber set forth in any one of [1] to [9], wherein the oily component comprises chamomile extract and cetyl 1,3-dimethylbutyl ether.
[16] The nanofiber set forth in any one of [1] to [9], wherein the oily component contains silicone oil.
[17] The nanofiber set forth in any one of [1] to [16], containing a volatile functional agent having a vapor pressure of 13.3 Pa or less at 20°C.
[18] A nanofiber sheet containing the nanofiber set forth in any one of [1] to [17].
[19] The nanofiber sheet set forth in [18], which is for use as a moisturizing sheet, a cosmetic sheet, or a medical sheet.
[20] A method for producing the nanofiber set forth in [1], comprising electrospinning an O/W emulsion having a water soluble polymer dissolved in an aqueous phase and an oily component in an oily phase.
[21] A method for producing the nanofiber set forth in [1], comprising providing a first liquid having a water soluble polymer dissolved in water and a second liquid containing an oily component and
   electrospinning the first liquid and the second liquid using a duplex-tube capillary having a sheath and a core,
   the first liquid being fed to the sheath, and the second liquid being fed to the core.
[22] The method set forth in [21], wherein the second liquid is prepared by emulsification.
[23] The method set forth in [21], wherein the second liquid is prepared by emulsification using a surfactant as an emulsifier.
[24] The method set forth in [23], wherein the emulsifier is a nonionic surfactant.
[25] The method set forth in [23], wherein the emulsifier is a polyethylene glycol monoalkylate, polyethylene glycol dialkylate, ethylene glycol dialkylate, or polyoxyethylene hydrogenated castor oil.
[26] The method set forth in [23], wherein the emulsifier is polyoxyethylene hydrogenated castor oil.
[27] A method for producing a nanofiber made from a water soluble polymer, containing an easily volatile functional agent having a vapor pressure higher than 13.3 Pa at 20°C, having a cavity, and containing an oily component in the cavity,
   the method comprising a step of applying the easily volatile functional agent to a layer of the nanofiber.
[28] A method for producing a nanofiber made from a water soluble polymer, containing an easily volatile functional agent having a vapor pressure higher than 13.3 Pa at 20°C, having a cavity, and containing an oily component in the cavity,
   the method comprising a step of disposing the easily volatile functional agent close to the nanofiber to cause the volatile functional agent to transfer to the nanofiber.

### Advantageous Effects of Invention

The invention provides a nanofiber that contains an oily component in a high ratio and yet has a reduced sticky feel caused by the oily component and also exhibits excellent storage stability.

### Brief Description of Drawings

[Fig. 1] Fig. 1(a), Fig. 1(b), Fig. 1(c), and Fig. 1(d) each represent a schematic cross-sectional structure of the nanofiber of the invention (Fig. 1(a), Fig. 1(b) and Fig. 1(d)) and of a reference fiber (Fig. 1(c)).
[Fig. 2] Fig. 2 is a schematic view of an apparatus suited to carry out electrospinning.
[Fig. 3] Fig. 3 is an enlarged schematic view showing the structure of the capillary used in the apparatus of Fig. 2.
[Fig. 4] Fig. 4(a) is a scanning electron micrograph of the nanofiber sheet obtained in Example 1, and Fig. 4(b) is a fluorescent micrograph of the nanofiber sheet obtained in Example 1.
[Fig. 5] Fig. 5(a) is a backscattered electron image of the nanofiber sheet obtained in Example 2, and Fig. 5(b) and Fig. 5(c) are X-ray elemental maps of silicon and carbon, respectively, of the nanofiber sheet obtained in Example 2.
[Fig. 6] Fig. 6 is a scanning electron micrograph of the nanofiber sheet obtained in Example 3.
[Fig. 7] Fig. 7 is a scanning electron micrograph of the nanofiber sheet obtained in Example 4.

### Description of Embodiments

The present invention provides a nanofiber containing a functional component in a high concentration and yet feeling dry and with the functional component being prevented from deteriorating. The invention also provides a nanofiber containing a functional component in its cavity and designed to slowly release the functional component to the skin by dissolving the outer water soluble polymer.

The invention will be described based on its preferred embodiments with reference to the accompanying drawings. The nanofiber of the invention has a thickness usually of 10 to 3000 nm, preferably 100 to 2000 nm, more preferably 200 to 1500 nm, in terms of circle equivalent diameter. The thickness of nanofibers is measured by, for example, observation using a scanning electron microscope (SEM). Specifically, a two-dimensional micrograph at a magnification of 10000 is processed by deleting defects (lumps of nanofibers, intersections of nanofibers, and polymer droplets), randomly choosing ten fibers, drawing a line perpendicular to the longitudinal direction of each fiber chosen, and reading the fiber diameter directly from the line. The length of the nanofiber is not critical in the invention and may have any appropriate length depending on the method of nanofiber manufacturing and the intended use of the nanofiber.

The nanofiber is made from a water soluble polymer. As used herein, the term "water soluble polymer" denotes a polymer having solubility such that, when a sample polymer weighing 1 g is immersed in 10 g of ion-exchanged water for 24 hours at 23°C and atmospheric pressure, at least 0.5 g of the immersed polymer dissolves in the ion-exchanged water.

Examples of the water soluble polymer include naturally occurring polymers, such as mucopolysaccharides, e.g., pullulan, hyaluronic acid, chondroitin sulfate, poly-γ-glutamic acid, modified corn starch, β-glucan, gluco-oligosaccharides, heparin, and keratosulfate, pectin, xylan, glucomannan, galacturonic acid, psyllium seed gum, tamarind seed gum, gum arabic, tragacanth gum, soybean water-soluble polysaccharides, alginic acid, carrageenan, laminaran, agar (agarose), fucoidan, methyl cellulose, hydroxypropyl cellulose, and hydroxypropylmethyl cellulose; and synthetic polymers, such as partially saponified polyvinyl alcohol, low-saponified polyvinyl alcohol, polyvinylpyrrolidone, polyethylene oxide, and sodium polyacrylate. These water soluble polymers may be used either individually or in combination of two or more thereof. Preferred of them are pullulan and synthetic polymers, such as partially saponified polyvinyl alcohol, low-saponified polyvinyl alcohol, polyvinylpyrrolidone, and polyethylene oxide, in view of ease of conversion to nanofibers.

The nanofiber has a cavity. The cavity provides a nanospace capable of holding an oily component described later. Figs: 1(a) through 1(d) each show a schematic cross-section of a nanofiber having a cavity.

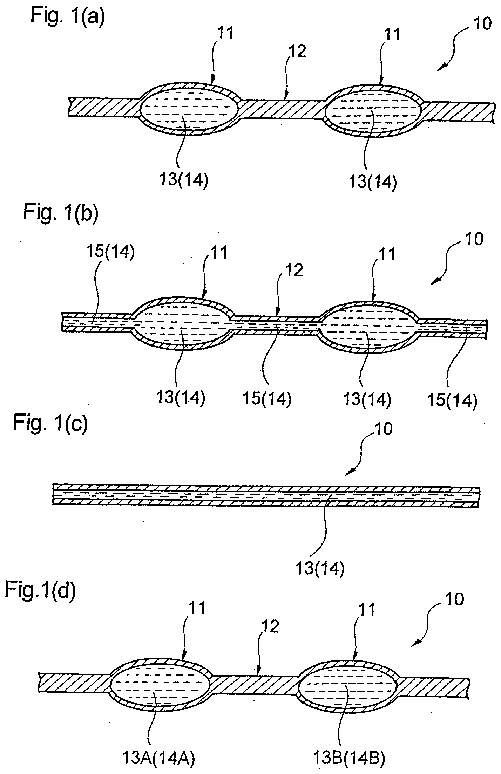

The nanofiber 10 shown in Fig. 1(a) has large-diametered portions 11 and small-diametered portions 12 alternating in the extending direction of the nanofiber 10. The large-diametered portion 11 has a cavity 13 having a nanospace inside. As above noted, the cavity 13 holds an oily component 14. On the other hand, the small-diametered portion 12 is solid, having no cavity.

While in Fig. 1(a) the large-diametered, portion 11 and the small-diametered portion 12 are depicted as being almost equal in length in the extending direction of the nanofiber 10, the length relation between them is not limited thereto. When the nanofiber 10 of the invention is produced by an electrospinning process described infra, the ratio of the length of the large-diametered portion to that of the small-diametered portion depends on the first liquid to second liquid ratio described infra. While in Fig. 1(a) the large-diametered portion 11 has a generally elliptic cross-section, the cross-sectional shape of the large-diametered portion is not limited thereto. A plurality of the large-diametered portions 11 may be equal or different in cross-sectional shape or thickness. While the small-diametered portion 12 is depicted as being constant in thickness, the thickness may be varied provided that it is smaller than that of the large-diametered portion 11. A plurality of the small-diametered portions 12 may be equal or different in thickness.

Similarly to the nanofiber of Fig. 1(a), the nanofiber 10 shown in Fig. 1(b) has large-diametered portions 11 and small-diametered portions 12. The difference of the nanofiber 10 of this embodiment from the nanofiber of Fig. 1(a) consists in the structure of the small-diametered portions. While the small-diametered portion of the nanofiber 10 of Fig. 1(a) is solid, the small-diametered portion 12 of the nanofiber of the present embodiment is tubular, forming a cavity 15. Similarly to the cavity 13 of the large-diametered portion 11, the cavity 15 of the small-diametered portion 12 holds an oily component 14. The cavity 13 of the large-diametered portion 11 and the cavity 15 of the adjacent small-diametered portion 12 are interconnnected. Not every cavity 13 of the large-diametered portion 11 of the nanofiber 10 needs to connect to the cavity 15 of the adjacent small-diametered portion 12.

The nanofiber 10 shown in Fig. 1(c) which is not subject of the present invention has no large-diametered portions unlike the nanofibers of Figs. 1(a) and 1(b). The nanofiber 10 shown has a tubular shape with a
substantially constant outer diameter and a substantially constant inner diameter over the whole length thereof, providing a tubular cavity 13 extending over the whole length thereof. The cavity 13 may be continuous or discontinuous over the whole length of the nanofiber 10. The tubular nanofiber 10 with substantially constant outer and inner diameters is able to contain in its cavity an increased amount of an oily component.

According to each of the above described embodiments, because the nanofiber 10 is capable of retaining an oily component 14 in its cavity 13, it is possible to incorporate the oily component 14 in a high ratio into the nanofiber 10. In particular, the nanofibers 10 of the embodiments shown in Figs. 1(b) and 1(c) are capable of containing the oily component 14 in a higher ratio because of the cavity 13 extending over the total length thereof. There is substantially no or, if any, very little oily component 14 on the surface of the nanofiber 10. Therefore, it is less likely for the nanofiber 10 to feel sticky due to the oily component 14. That is, even when in using an oily component that is liquid at ambient temperature (5° to 35°C), the nanofiber 10 is able to contain such an oily component in a high ratio while maintaining a dry feel.

The oily component may be used either alone or in the form of a solution in an organic solvent. Accordingly, the cavity 13 may contain the oily component alone or a solution of the oily component in an organic solvent.

As stated, the nanofiber 10 of the invention is capable of containing an oily component in a higher ratio than conventional nanofibers, preferably in a ratio of 0.5% to 95%, more preferably 10% to 90%, even more preferably 20% to 90%, by mass. The ratio of the water soluble polymer in the nanofiber 10 is preferably 5% to 99.5%, more preferably 10% to 90%, even more preferably 10% to 80%, by mass. The ratios of the water soluble polymer and the oily component in the nanofiber 10 are determined by dissolving a given amount of a nanofiber sheet in water and subjecting the solution to centrifugation.

A variety of oily components are usable in accordance with the intended use of the nanofiber 10. The oily component that can be used for use in, for example, a moisturizing sheet, a cosmetic sheet, or a medical sheet contains a solvent selected from squalane, olive oil, silicone oil, macadamia nut oil, or cetyl 1,3-dimethylbutyl ether, and an oil-soluble component generally used in cosmetics or for medical use selected from vitamin E, chamomile extract, or rose extract, as dissolved in the solvent as an active ingredient.

The oily components described above may be used either individually or in combination of two or more thereof. In the latter case, when a nanofiber having, for example, the cross-sectional structure shown in Fig. 1(a) is produced using a first oily component and a second oily component, both the first and the second oily component may be present in each cavity 13, or they may be present according to the embodiment shown in Fig. 1(d). In the embodiment of Fig. 1(d), some cavities 13A of the nanofiber 10 contain the first oily component and do not contain the second oily component, and other cavities 13B contain the second oily component and do not contain the first oily component.

The nanofiber 10 of the invention may further contain a volatile functional agent in addition to the above described components. The volatile functional agent is preferably at least one member selected from the group consisting of a fragrance, a whitening agent, and a taste corrector. As used herein, the "fragrance" means a substance capable of imparting a pleasant scent to air at ambient temperature and atmospheric pressure and having a "fragrant function". As used herein, the "whitening agent" means a substance which is, when applied to human skin, capable of whitening the skin or maintaining the skin in a youthful and healthy condition and has a "whitening function". As sued herein, the "taste corrector" means a substance capable of changing or diminishing a taste and having a "taste correcting function". For example, a taste corrector may change bitterness or sourness to another taste, e.g., sweetness, or reducing a taste. These functional agents are volatile substances vaporizing at ambient temperature and atmospheric pressure. As used herein, the term "ambient temperature and atmospheric pressure" usually means a condition of a temperature of 23°C and an atmospheric pressure of 101.325 kPa. When the volatile functional agent is water soluble, it exists along with the water soluble polymer. When it is oil soluble, it exists in the same cavity as the above discussed oily component or, as shown in Fig. 1(d), in a cavity different from the cavity containing the oily component. Irrespective of whether the volatile functional agent is water soluble or oil soluble, it is possible to locate the volatile functional agent on or in the vicinity of the surface of a nanofiber.

The volatile functional agent preferably has a vapor pressure at 20°C of 13.3 Pa or less, more preferably 0.0013 to 10.7 Pa, even more preferably 0.0133 to 6.7 Pa. With the vapor pressure at 20°C of the volatile functional agent falling within that range, the nanofiber 10 will exhibit a useful function attributed to the volatile functional agent at ambient temperature and atmospheric pressure. For instance, a nanofiber 10 containing a fragrance as a volatile functional agent is a fragranced nanofiber capable of releasing a fragrance into air at ambient temperature and atmospheric pressure thereby to make a user feel exhilarated, refreshed, cleaned, or relaxed and also produce a deodorizing effect, an anesthetic (analgesic) effect, or a like effect.

When the nanofiber is produced by electrospinning (a method for producing the nanofiber, hereinafter described) using a stock polymer solution containing a volatile functional agent whose vapor pressure at 20°C exceeds 13.3 Pa (hereinafter also referred to as an easily volatile functional agent), the easily volatile functional agent tends to vaporize during electrospinning because of its high volatility. It can follow that the resulting nanofiber fails to retain a sufficient amount of the easily volatile functional agent for performing the function expected of the use of the functional agent, for example, a fragrant function of an easily volatile fragrance. Nevertheless, it is possible to impart the function of such an easily volatile functional agent to a nanofiber by timely adding the easily volatile functional agent (see methods A and B described infra). The vapor pressure of a volatile functional agent is obtained from the data base provided by Research Institute for Fragrance Materials.

Examples of fragrances that can be used as a volatile functional agent include vanillin, methyl jasmonate, γ-undecalactone, and phenylethyl alcohol. These volatile functional agents may be used either individually or in combination of two or more thereof.

The volatile functional agent content in the nanofiber 10 is preferably 0.001% to 30%, more preferably 0.01% to 5%, by mass. The recited range of the volatile functional agent content assures production of a nanofiber performing a useful function of the volatile functional agent, such as fragrance release, and achieves reduction in the amount of the volatile functional agent to be used, leading to a reduced cost of production.

The nanofiber 10 of the invention is used to advantage in the form of a sheet that contains the nanofiber 10 of the invention. A sheet containing the nanofiber 10 of the invention (hereinafter called a "nanofiber sheet") may be made solely of the nanofiber 10 or may contain other fibers, such as nanofibers except the nanofiber 10 of the invention and ordinary natural or synthetic fibers. Also included in the nanofiber sheet of the invention is a laminate sheet composed of a fiber sheet containing the nanofiber 10 of the invention and at least one other fiber sheet and/or film.

The nanofibers in the nanofiber sheet are bonded to one another at the intersections thereof or intertwined with one another, whereby the nanofiber sheet is self-supporting. Whether the nanofibers are bonded to or intertwined with one another depends on the method of manufacture.

The nanofiber sheet may have a thickness decided as appropriate to the intended use. For example, for use as attached to human skin, the thickness of the nanofiber sheet is preferably 50 nm to 1 mm, more preferably 500 nm to 500 µm. The thickness of the nanofiber sheet may be measured using a contact thickness gauge Litematic VL-50A from Mitutoyo Corp.

The nanofiber sheet of the invention is used as attached to, for example, human skin, the skin of mammals other than human, tooth, and surfaces of plant parts, such as foliage. Before the nanofiber sheet is applied to the surface of an object, a surface of the nanofiber sheet or the surface of the object is wetted with water or a water-containing aqueous liquid so that the nanofiber sheet successfully adheres to the surface with the surface tension taken advantage of In addition, the water soluble polymer making up the nanofiber dissolves in the liquid. It follows that the cavity 13 is destroyed to make the oily component retained therein flow out. The flowing-out oily component covers the surface of the object such as human skin and penetrates inside the object. When, for example, a nanofiber sheet containing a plant extract as an oily component is applied to human skin, the oily component having flowed out from the destroyed cavity covers the skin surface and penetrates inside the skin to achieve its action and effect.

It is particularly advantageous that the oily component covering the surface of the object, e.g., human skin inhibits evaporation of water from the object to easily keep the surface of the object moisturized. This is expected to lead to the following effects. The functional agent, if present in the nanofiber, is helped to penetrate inside the object. The oily component effectively prevents the water soluble polymer making up the nanofiber from drying and filming, so that an uncomfortable pulling sensation that might be caused by the filming is reduced. The oily component acts as a plasticizer on the water soluble polymer film to maintain the transparency and impart flexibility to the water soluble polymer film. Uncomfortable powdery feel that might be caused by partial peeling of the water soluble polymer film and poor appearance that might be caused by whitening due to partial lifting of water soluble polymer film is reduced.

The surface of an object or the surface of the nanofiber sheet may be wetted by, for example, spreading or spraying a liquid of various kinds to the surface. The liquid to be spread or sprayed is a substance containing water and having a viscosity of about 5000 mPa·s or less at the use temperature. Such a liquid is exemplified by water, an aqueous solution, or an aqueous dispersion. Emulsions, including O/W emulsions and W/O emulsions, and aqueous liquids thickened with a thickener are also useful. More specifically, in the case when the nanofiber sheet is attached to human skin, a skin lotion or a beauty cream is useful as a liquid wetting the surface of the object (skin).

In order to wet the surface of an object or the surface of the nanofiber sheet by spreading or spraying a liquid, it suffices that the liquid be applied in a minimum amount required for the liquid to sufficiently exhibit a surface tension and to dissolve the water soluble polymer. The amount of a liquid to be applied varies with the size of the nanofiber sheet. For example, in the case of a nanofiber sheet measuring 3 cm by 3 cm, presence of about 0.01 ml of a liquid on the surface of an object will be enough to attach the nanofiber sheet to the object easily and to dissolve the water soluble polymer of the nanofiber 10 to destroy the cavity 13.

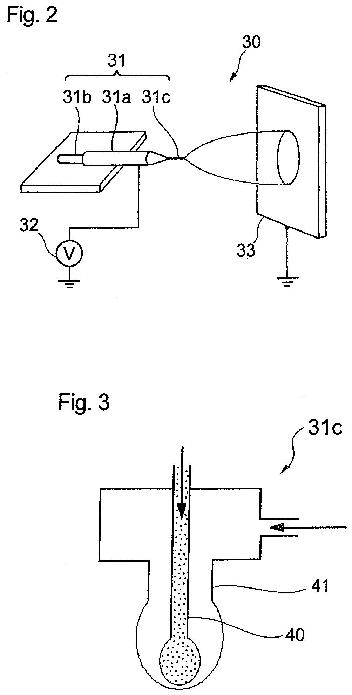

The above described nanofiber sheet is suitably produced by, for example, electrospinning deposition (ESD). Fig. 2 illustrates an apparatus 30 for carrying out ESD. The apparatus 30 includes a syringe 31, a high voltage supply 32, and a conductive collector 33. The syringe 31 has a cylinder 31a, a plunger 31b, and a capillary 31c. The capillary 31c has an inner diameter of about 10 to 1000 µm. The cylinder 31a is filled with a stock solution, the raw material of a nanofiber. The high voltage supply 32 is, for example, a 10 to 30 kV direct voltage source. The positive pole of the high voltage supply 32 is electrically connected to the stock solution in the syringe 31, with the negative pole grounded. The conductive collector 33 is, e.g., a metal plate that is grounded. The distance between the tip of the capillary 31c of the syringe 31 and the conductive collector 33 is set at, e.g., about 30 to 300 mm. The amount of the stock solution ejected from the capillary 31c is preferably 0.1 to 10 ml/hr, more preferably 0.1 to 4 ml/hr. The apparatus 30 shown in Fig. 2 may be operated in the atmosphere. The operative environment is not particularly limited and may be, for example, 20° to 40°C and 10% to 50% RH.

Preparation of the stock solution is of importance in order to successfully produce the nanofiber 10 having the cavity 13. In the case of obtaining the nanofiber 10 of the embodiment shown in Figs. 1(a) or 1(b), the stock solution is prepared by mixing a first and a second liquid. The first liquid is an aqueous solution of a water soluble polymer in water, and the second liquid is an O/W emulsion having an oily component dispersed in an aqueous phase. On mixing the two liquids, there is prepared an O/W emulsion having the water soluble polymer dissolved in the aqueous phase and the oily component contained in the oily phase. The thus prepared O/W emulsion is used as a stock solution to be electrospun to produce the nanofiber 10 having a desired configuration and a nanofiber sheet.

The first liquid preferably has a water soluble polymer concentration of 3% to 30%, more preferably 10% to 25%, by mass, in view of suitable viscosity of the resulting stock solution. The first liquid is obtained by adding a water soluble polymer to water or an aqueous liquid containing a small amount of a water soluble organic solvent and mixing them with or without heating.

The second liquid is obtained by a known emulsifying technique, such as spontaneous emulsification, phase-transfer emulsification, or forced emulsification. The mass ratio of the aqueous phase to the oily phase containing the oily component, aqueous phase/oily phase, is preferably 51:49 to 99:1, more preferably 51:49 to 85:15, to successfully accomplish emulsification. For the same reason, the amount of an emulsifier to be used for emulsification is preferably 0.001% to 20%, more preferably 0.004 to 7%, by mass based on the total mass of the first and the second liquid.

Various surfactants can be used as an emulsifier. Nonionic surfactants, such as a polyethylene glycol monoalkylate, a polyethylene glycol dialkylate, an ethylene glycol dialkylate, and polyoxyethylene hydrogenated castor oil, are particularly preferred for their low irritation to the skin.

When the second liquid is prepared by phase transfer emulsification, an emulsifier is added to an oily phase containing an oily component, followed by heating to a prescribed temperature, and an aqueous phase heated to a prescribed temperature is slowly added thereto while stirring to induce phase transfer to yield an O/W emulsion.

The resulting O/W emulsion (stock solution) is preferably composed of 55% to 98%, more preferably 60% to 97%, by mass of the aqueous phase and 2% to 45%, more preferably 3% to 40%, by mass of the oily phase.

In the cases of using two or more oily components, the following method (I) or (II) may be employed to prepare the stock solution.
Method (I): An O/W emulsion containing all the oily components in its oily phase is prepared as a second liquid, which is mixed with a first liquid to prepare a stock solution.
Method (II): An O/W emulsion is prepared for each oily component, and all the resulting O/W emulsions are mixed with a first liquid to prepare a stock solution.

When the method (I) is followed to produce the nanofiber shown in Fig. 1(a), all the oily components are present in every cavity 13. When the method (II) is used to produce the nanofiber shown in Fig. 1(a), some cavities contain only the first oily component and no other oily components, and some other cavities contain only the second oily component and no other oily components (see Fig. 1(d)).

Electrospinning using the O/W emulsion as a stock solution results in the formation of the nanofiber 10 having the structure shown in Figs. 1(a) or 1(b). This is because, the inventors believe, upon jetting the stock solution, the phase of the water soluble polymer solution containing much water that is a volatile component tends to exist in the outermost layer, whereas the phase of the oily component undergoing little solvent vaporization tends to exist inside.

To produce the nanofiber 10 having the structure shown in Fig. 1(c), a solution of a water soluble polymer in water is used as a first liquid, and an oily component or a solution of an oily component in an organic solvent is used as a second liquid. Electrospinning is carried out using the apparatus of Fig. 2 in which the capillary 31c is a duplex-tube capillary having an inner cylinder 40 and an outer cylinder 41 as shown in Fig. 3. The second liquid is fed to the core, and the first liquid is fed to the sheath. A nanofiber having the structure as designed is obtained successfully by properly balancing the amounts of the first and the second liquid to be ejected.

When the above discussed volatile functional agent which is water soluble is used, it is incorporated into the first liquid. The functional agent will then exist together with the water soluble polymer in the resulting nanofiber. When the volatile functional agent which is oil soluble is used, on the other hand, the following methods (i) or (ii) may be employed.
Method (i): An O/W emulsion containing an oily component and the volatile functional agent in its oily phase is prepared as a second liquid, which is then mixed with a first liquid to prepare a stock solution.
Method (ii): Separately from an O/W emulsion containing an oily component in its oily phase as a second liquid, an O/W emulsion containing the volatile functional agent in its oily phase is prepared as a third liquid. The second and the third liquid are mixed with a first liquid to prepare a stock solution. In this method, the second liquid does not contain the volatile functional agent, and the third liquid does not contain the oily component.

When the method (i) is adapted to produce the nanofiber shown in Fig. 1(a), the oily component and the volatile functional agent are present in every cavity 13. When the method (ii) is followed to produce the nanofiber shown in Fig. 1(a), some cavities contain the oily component but does not contain the volatile functional agent, and some other cavities contain the volatile functional agent but does not contain the oily component (see Fig. 1(d)).

In the case when a highly volatile functional agent, for example, the easily volatile functional agent discussed earlier is used, the easily volatile functional agent is allowed to be imparted to a nanofiber by timely addition of the easily volatile functional agent. More specifically, a nanofiber provided with the function of an easily volatile functional agent is obtainable according to the following methods A and B.

Method A: A method for producing a nanofiber including a step of making a nanofiber having a cavity according to the above described method (nanofiber making step) and a step of applying a solution containing an easily volatile functional agent to the nanofiber (solution addition step).

Method B: A method for producing a nanofiber including a step of making a nanofiber having a cavity according to the above described method (nanofiber making step) and a step of leaving an easily volatile functional agent to stand close to the nanofiber for a prescribed period of time (easily volatile functional agent transfer step).

The solution containing an easily volatile functional agent for use in the solution addition step of the method A is prepared by dissolving or dispersing the easily volatile component in a solvent. The solvent is preferably uninfluential on the nanofiber, specifically the water soluble polymer making up the nanofiber. An organic solvent may be used, for example. Application of the solution containing the easily volatile functional agent to the nanofiber may be achieved by, for example, spraying the solution to the nanofiber or immersing the nanofiber in the solution.

The easily volatile functional agent transfer step of the method B is a step in which the nanofiber and the easily volatile functional agent are brought close to but not in contact with each other thereby to cause the vapor of the easily volatile functional agent to transfer to the nanofiber. If the nanofiber and the easily volatile functional agent are brought into contact with each other, the water soluble polymer constituting the nanofiber can dissolve or swell to lose its form. Such an inconvenience is avoided by placing them close to each other. The easily volatile functional agent placed close to the nanofiber may be exposed to open air or be enclosed in an air permeable bag or a like enclosure. Unintentional direct contact of the nanofiber with the easily volatile functional agent is certainly avoided by enclosing the easily volatile functional agent. The period of time that the easily volatile functional agent is left to stand close the nanofiber is decided as appropriate to the type of the easily volatile functional agent and the like. In general, the higher the volatility of the functional agent, the shorter the time needed.

While the invention has been described with reference to its preferred embodiments, it should be understood that the invention is not limited to these embodiments. For example, while the method for producing the nanofiber has been described with particular reference to electrospinning deposition, the method for producing the nanofiber is not limited thereto.

While, according to the electrospinning technique shown in Fig. 2, the nanofiber formed is deposited on the conductive collector 33 of plate shape, a conductive rotating drum may be used instead of the plate-shaped collector, in which case the nanofiber is deposited on the peripheral surface of the rotating drum.

The invention will now be illustrated in greater detail by way of Examples, but it should be understood that the scope of the invention is not limited thereto. Unless otherwise noted, all the percents are by mass.

### Example 1

### (1) Preparation of first liquid

Pullulan available from Hayashibara Shoji Inc. was used as a water soluble polymer. Pullulan was dissolved in water to make a 20% aqueous solution as a first liquid. The first liquid was heated to 80°C.

### (2) Preparation of second liquid

A mixture of chamomile extract and cetyl 1,3-dimethylbutyl ether (ASE166K from Kao Corp.) was used as an oily component. The concentration of chamomile extract in the solution was 4.20%. Polyoxyethylene hydrogenated castor oil (Emanon® CH60, from Kao Corp.) as a nonionic surfactant was added to the solution in a concentration of 0.3%. A 0.95 ml portion of the resulting solution was heated to 80°C, and 4.00 ml of hot water at 80°C was slowly added thereto while stirring to cause phase-transfer emulsification, thereby to give an O/W emulsion as a second liquid.

### (3) Preparation of stock solution

The first liquid and the second liquid were mixed and stirred at a mass ratio of 3:1 to prepare an O/W emulsion as a stock solution. The stock solution was found to contain 15.00% of pullulan, 80.20% of water, 4.78% of the oily component, and 0.013% of the nonionic surfactant.

### (4) Electrospinning

The stock solution obtained above was electrospun under the conditions described below using the apparatus shown in Fig. 2 to form a nanofiber sheet on the surface of a 25 µm thick polyethylene terephthalate film disposed on the surface of the conductive collector 33.
Applied voltage: 25 kV
Capillary-collector distance: 185 mm
Rate of ejection of stock solution: 1 ml/hr
Environment: 25°C, 50% RH

### (5) Evaluation

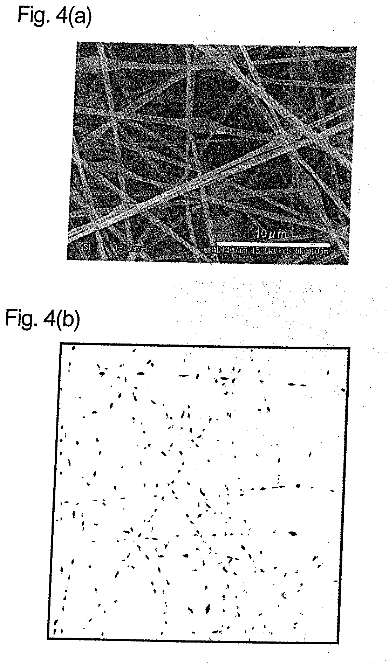

The resulting nanofiber sheet contained 75.82% of pullulan, 24.11% of the oily component, and 0.07% of the surfactant. The thickness of the nanofiber sheet was found to be 30 µm measured with Litematic VL-50A from Mitutoyo Corp. A scanning electron micrograph of the nanofiber sheet is shown in Fig. 4(a), from which the diameter of the small-diametered portion of the nanofiber was found to be 504 nm. Separately, electrospinning deposition was carried out in the same manner as described above, except for adding an oily fluorescent agent Nile Red to the second liquid. A fluorescent micrograph of the resulting nanofiber sheet is shown in Fig. 4(b), in which the black spots correspond to the portions containing the fluorescent agent.

As is apparent from Fig. 4(a), the nanofiber of the nanofiber sheet obtained in Example 1 has large-diametered portions and small-diametered portions. It is seen from Fig. 4(b) that the nanofiber has cavities each at the large-diametered portion and that the oily component is retained in the cavities. The nanofiber of Example 1 is thus proved to have the structure shown in Fig. 1(a).

### Example 2

### (1) Preparation of first liquid

The same as in Example 1.

### (2) Preparation of second liquid

Silicone oil was used as an oily component. Polyoxyethylene hydrogenated castor oil (Emanon® CH60, from Kao Corp.) as a nonionic surfactant was added to the silicone oil in a concentration of 0.3%. A 0.95 ml portion of the silicone oil was heated to 80°C, and 4.00 ml of hot water at 80°C was slowly added thereto while stirring to cause phase-transfer emulsification, thereby to give an O/W emulsion as a second liquid.

### (3) Preparation of stock solution

The first liquid and the second liquid were mixed and stirred at a mass ratio of 3:1 to prepare an O/W emulsion as a stock solution. The stock solution was found to contain 15% of pullulan, 80.2% of water, 4.787% of the oily component, and 0.013% of the nonionic surfactant.

### (4) Electrospinning

The same as in Example 1.

### (5) Evaluation

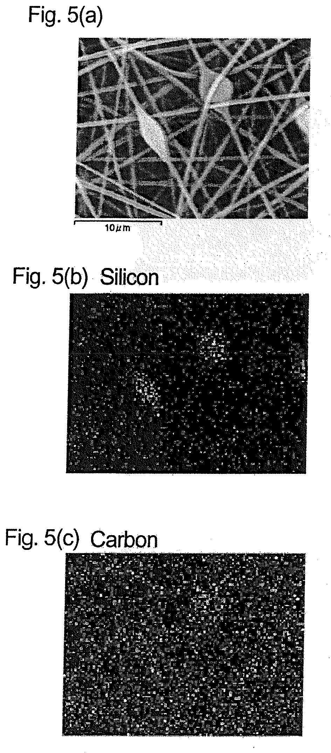

The resulting nanofiber sheet contained 75.82% of pullulan, 24.11% of the oily component, and 0.07% of the surfactant. The thickness of the nanofiber sheet was found to be 30 µm measured with Litematic VL-50A from Mitutoyo Corp. A backscattered electron image of the nanofiber sheet is shown in Fig. 5(a), from which the diameter of the small-diametered portion of the nanofiber was found to be 490 nm. The Si and C distribution in the observation field shown in Fig. 5(a) was analyzed by EDX elementary mapping. The results obtained are shown in Figs. 5(b) and 5(c). As is apparent from Fig. 5(a), the nanofiber obtained in Example 2 has large-diametered portions and small-diametered portions. As can be seen from Figs. 5(b) and 5(c), the large-diametered portions have the respective cavities containing the oily component. The nanofiber of Example 2 is thus proved to have the structure shown in Fig. 1(a).

### Example 3

### (1) Preparation of first liquid

The same as Example 1.

### (2) Preparation of second liquid

Chamomile extract was used as an oily component. Polyoxyethylene hydrogenated castor oil (Emanon® CH60, from Kao Corp.) as a nonionic surfactant was added to the solution in a concentration of 0.3%. A 1.72 ml portion of the resulting solution was heated to 80°C, and 2.06 ml of hot water at 80°C was slowly added thereto while stirring to cause phase-transfer emulsification, thereby to give an O/W emulsion as a second liquid.

### (3) Preparation of stock solution

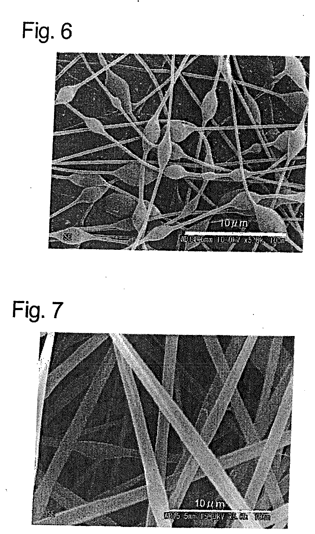

The first liquid and the second liquid were mixed and stirred at a mass ratio of 81:19 to prepare an O/W emulsion as a stock solution. The stock solution was found to contain 16.22% of pullulan, 75.16% of water, 8.59% of the oily component, and 0.03% of the nonionic surfactant. A nanofiber sheet was obtained in the same manner as in Example 1, except for using the resulting stock solution. The resulting nanofiber sheet contained 65.3% of pullulan, 34.58% of the oily component, and 0.12% of the surfactant. The thickness of the nanofiber sheet was found to be 30 µm measured with Litematic VL-50A from Mitutoyo Corp. A scanning electron micrograph of the nanofiber sheet is shown in Fig. 6. The Fig. 6 reveals that the nanofiber of the nanofiber sheet of Example 3 has large-diametered portions and small-diametered portions. The diameter of the small-diametered portions of the nanofiber was found to be 270 nm from the micrograph. Because the nanofibers of Example 3 have a higher ratio of the oily component and a shorter distance between adjacent large-diametered portions than the nanofibers of Fig. 4(a), it is considered that the small-diametered portions also have cavities in which the oily component is retained.

### Example 4 (Reference)

Pullulan from Hayashibara Shoji Inc. was used as a water soluble polymer. Pullulan was dissolved in water to make a 20% aqueous solution as a first liquid. Chamomile extract, an oily component, was used as a second liquid. Electrospinning was carried out using these liquids and the apparatus 30 shown in Fig. 2 under the conditions described below. The capillary 31c of the apparatus 30 had the structure of Fig. 3. The second liquid was fed to the core, and the first liquid to the sheath. A nanofiber sheet was obtained in otherwise the same manner as in Example 1.
Applied voltage: 25 kV
Capillary-collector distance: 220 mm
Rate of ejection of first liquid: 0.1 ml/hr
Rate of ejection of second liquid: 2 ml/hr
Environment: 25°C, 50% RH

The resulting nanofiber sheet contained 80% of pullulan and 20% of the oily component. The thickness of the nanofiber sheet was found to be 30 µm measured with Litematic VL-50A from Mitutoyo Corp. A scanning electron micrograph of the nanofiber sheet is shown in Fig. 7, from which the diameter of the nanofiber was found to be 1312 nm. Fig. 7 proves that the nanofiber of the nanofiber sheet obtained in Example 4 has the structure shown in Fig. 1(c).

### Comparative Example 1

Chamomile extract was added to a 15% pullulan solution in a ratio of pullulan 16.22%, water 75.17%, and oily component 8.59%, followed by stirring using a stirrer. However, chamomile extract and the pullulan solution separated from each other, resulting in a failure to provide a uniform solution. Therefore, electrospinning was not conducted.

### Comparative Example 2

The stock solution prepared in Example 1 was dropped on a petri dish and left to dry to give a 30 µm thick cast film.

### Comparative Example 3

The stock solution prepared in Example 2 was dropped on a petri dish and left to dry to give a 30 µm thick cast film.

### Comparative Example 4

The stock solution prepared in Example 3 was dropped on a petri dish and left to dry to give a 30 µm thick cast film.

### Evaluation for feel of use

Oil-blotting paper (from DHC Corp.) was applied to the nanofiber sheets obtained in Examples 1 to 4 and the cast films of Comparative Examples 2 to 4 to visually observe absorption of the oily component. More specifically, the oil-blotting paper was pressed onto the sheet or cast film. After removal from the sheet or film, the change in color of the oil-blotting paper was observed with naked eyes. Furthermore, dissolving properties of the sheets or cast films when attached to human skin were evaluated. The results of evaluation are shown in Table 1 below. The methods of evaluation are as follows.

### (1) Oily component absorption by oil-blotting paper

A square of about 3 cm per side was cut out of the sheet or cast film, and the oil-blotting paper (from DHC) was applied and removed. The change in color of the oil-blotting paper was observed with naked eyes.

### (2) Dissolving properties when attached to human skin

A skin lotion weighing 0.03 g was dropped on the skin and spread to a circle of about 20 mm diameter. A square of about 15 mm per side cut out of the sheet or cast film was put thereon. Immediately thereafter, the dissolved state of the sheet or cast film was observed with naked eyes and rated as follows.
A: The sheet or film immediately dissolves and becomes unable to be picked up with tweezers.
B: The sheet or film partially dissolves and partially remains in sheet form that is able to be picked up with tweezers.

**Table 1**

| | Stability of Solution | Absorption of Oily Component by Oil-Blotting Paper | Dissolving Properties when Attached to Human Skin |
|---|---|---|---|
| Example 1 | OK | no | A |
| Example 2 | OK | no | A |
| Example 3 | OK | no | A |
| Example 4 | OK | no | A |
| Comp. Example 1 | NG | - | - |
| Comp. Example 2 | OK | yes | B |
| Comp. Example 3 | OK | yes | B |
| Comp. Example 4 | OK | yes | B |

As is apparent from the results in Table 1, it is seen that the nanofiber sheets of Examples have a reduced sticky feel because the oily component is not exposed on the surface of the fiber and that they easily dissolve on contact with water to release the oily component.

## Claims

1. A nanofiber comprising a water soluble polymer, having a cavity, and containing an oily component in the cavity,
the nanofiber having a large-diametered portion and a small-diametered portion, and
the large-diametered portion having the cavity wherein the oily component contains a solvent selected from squalane, olive oil, silicone oil, macadamia nut oil, or cetyl 1,3-dimethylbutyl ether, and an oil-soluble component selected from vitamin E, chamomile extract, or rose extract, as dissolved in the solvent as an active ingredient.

2. The nanofiber according to claim 1, wherein the small-diametered portion has the cavity, and the cavity in the large-diametered portion and the cavity in the small-diametered portion are interconnected.

3. The nanofiber according to claim 1 or 2, wherein the water soluble polymer is one of, or a combination of two or more of pullulan or a synthetic polymer selected from the group consisting of partially saponified polyvinyl alcohol, low-saponified polyvinyl alcohol, polyvinylpyrrolidone, and polyethylene oxide.

4. The nanofiber according to any one of claims 1 to 3, wherein the oily component comprises chamomile extract, cetyl 1,3-dimethylbutyl ether or silicone oil.

5. The nanofiber according to any one of claims 1 to 4, wherein the oily component comprises a first oily component and a second oily component,
the cavity comprises a plurality of cavities discretely formed over the whole length of the nanofiber, and
the cavities contain both the first oily component and the second oily component.

6. The nanofiber according to any one of claims 1 to 5, containing a volatile functional agent having a vapor pressure of 13.3 Pa or less at 20°C.

7. A nanofiber sheet comprising the nanofiber according to any one of claims 1 to 6.

8. The nanofiber sheet according to claim 7, having a surface which is to be attached to human skin, or the skin or tooth of mammals other than human.

9. The nanofiber sheet according to claim 8, which is for use as a sheet which is to be attached to human skin, and has a thickness of 50 nm to 1 mm.

10. Use of the nanofiber sheet according to any one of claims 7 to 9 as a moisturizing sheet, a cosmetic sheet, or a medical sheet.

11. A method for producing the nanofiber according to claim 1, comprising electrospinning an O/W emulsion having a water soluble polymer dissolved in an aqueous phase and an oily component contained in an oily phase.

12. The method for producing the nanofiber according to claim 11, wherein the O/W emulsion is prepared by mixing a first liquid which is an aqueous solution of a water soluble polymer dissolved in water and a second liquid which is an O/W emulsion having an oily component contained in an aqueous phase.

13. The method for producing the nanofiber according to claim 12, wherein the O/W emulsion of the second liquid is prepared by using a nonionic surfactant as an emulsifier.

14. A method for keeping a surface of an object moisturized by wetting the surface of the object with water or a water-containing aqueous liquid before applying the nanofiber sheet according to claims 7 to 9 to the surface of the object thereby destroying the cavity of the nanofiber so that the oily component retained in the cavity flows out and the flowing-out oily component covers the surface of the object wherein the object is human skin.

## Patentansprüche

1. Eine Nanofaser, umfassend ein wasserlösliches Polymer mit einem Hohlraum und eine ölige Komponente, die in dem Hohlraum enthalten ist,
wobei die Nanofaser einen Bereich mit großem Durchmesser und einen Bereich mit kleinem Durchmesser aufweist und,
wobei der Bereich mit großem Durchmesser den Hohlraum aufweist, wobei die ölige Komponente ein Lösungsmittel, ausgewählt aus Squalan, Olivenöl, Silikonöl, Macadamianussöl oder Cetyl-1,3-dimethylbutyl-ether, und eine öllösliche Komponente, ausgewählt aus Vitamin E, Kamillenextrakt oder Rosenextrakt, die in dem Lösungsmittel als Wirkstoff gelöst ist, enthält.

2. Die Nanofaser gemäß Anspruch 1, wobei der Bereich mit kleinem Durchmesser den Hohlraum aufweist und der Hohlraum in dem Bereich mit großem Durchmesser und der Hohlraum in dem Bereich mit kleinem Durchmesser miteinander verbunden sind.

3. Die Nanofaser gemäß Anspruch 1 oder 2, wobei das wasserlösliche Polymer eines von, oder eine Kombination aus zwei oder mehreren von Pullulan oder einem synthetischen Polymer, ausgewählt aus der Gruppe bestehend aus teilweise verseiftem Polyvinylalkohol, niedrig verseiftem Polyvinylalkohol, Polyvinylpyrrolidon und Polyethylenoxid, ist.

4. Die Nanofaser gemäß einem der Ansprüche 1 bis 3, wobei die ölige Komponente Kamillenextrakt, Cetyl-1,3-dimethylbutyl-ether oder Silikonöl umfasst.

5. Die Nanofaser gemäß einem der Ansprüche 1 bis 4, wobei die ölige Komponente eine erste ölige Komponente und eine zweite ölige Komponente umfasst,
wobei der Hohlraum eine Vielzahl an Hohlräumen, die über die gesamte Länge diskret gebildet sind, umfasst und,
wobei die Hohlräume sowohl die erste ölige Komponente als auch die zweite ölige Komponente enthalten.

6. Die Nanofaser gemäß einem der Ansprüche 1 bis 5, die ein flüchtiges funktionelles Mittel mit einem Dampfdruck von 13,3 Pa oder weniger bei 20°C enthält.

7. Ein Nanofaserbahnenmaterial, umfassend die Nanofaser gemäß einem der Ansprüche 1 bis 6.

8. Das Nanofaserbahnenmaterial gemäß Anspruch 7, das eine Oberfläche aufweist, zur Anbringung an die menschliche Haut oder die Haut oder den Zahn von Säugetieren, ausgenommen Menschen.

9. Das Nanofaserbahnenmaterial gemäß Anspruch 8 zur Verwendung als Bahnenmaterial, das an die menschliche Haut angebracht werden soll, und das eine Dicke von 50 nm bis 1 mm aufweist.

10. Verwendung des Nanofaserbahnenmaterials gemäß einem der Ansprüche 7 bis 9 als ein feuchtigkeitsspendendes Bahnenmaterial, ein kosmetisches Bahnenmaterial oder ein medizinisches Bahnenmaterial.

11. Ein Verfahren zur Herstellung der Nanofaser gemäß Anspruch 1, umfassend das Elektrospinnen einer Ö/W-Emulsion, die ein wasserlösliches Polymer, das in einer wässrigen Phase gelöst ist und eine ölige Komponente, die in einer öligen Phase enthalten ist, aufweist.

12. Das Verfahren zur Herstellung der Nanofaser gemäß Anspruch 11, wobei die Ö/W-Emulsion durch Mischen einer ersten Flüssigkeit, die eine wässrige Lösung eines wasserlöslichen Polymers, das in Wasser gelöst ist, ist und einer zweiten Flüssigkeit, die eine Ö/W-Emulsion mit einer öligen Komponente, die in einer wässrigen Phase enthalten ist, ist, hergestellt wird.

13. Das Verfahren zur Herstellung der Nanofaser gemäß Anspruch 12, wobei die Ö/W-Emulsion der zweiten Flüssigkeit unter Verwendung eines nichtionischen oberflächenaktiven Mittels als Emulgierungsmittel hergestellt wird.

14. Ein Verfahren zur Feuchthaltung einer Oberfläche eines Objekts, durch Befeuchten der Oberfläche des Objekts mit Wasser oder einer wasserhaltigen wässrigen Flüssigkeit vor Anbringen des Nanofaserbahnenmaterials gemäß einem der Ansprüche 7 bis 9 auf die Oberfläche des Objekts, wodurch der Hohlraum der Nanofaser zerstört wird, sodass die ölige Komponente, die sich in dem Hohlraum befand, herausfließt und die herausfließende ölige Komponente die Oberfläche des Objekts bedeckt, wobei das Objekt menschliche Haut ist.

## Revendications

1. Nanofibre comprenant un polymère hydrosoluble, ayant une cavité, et contenant un composant huileux dans la cavité,
la nanofibre ayant une portion de gros diamètre et une portion de petit diamètre, et
la portion de gros diamètre ayant la cavité dans laquelle le composant huileux contient un solvant choisi parmi le squalane, l'huile d'olive, l'huile siliconée, l'huile de noix de macadamia, ou le cétyl-1,3-diméthylbutyléther, et un composant liposoluble choisi parmi la vitamine E, l'extrait de camomille, ou l'extrait de rose, sous forme dissoute dans le solvant comme ingrédient actif.

2. Nanofibre selon la revendication 1, dans laquelle la portion de petit diamètre renferme la cavité, et la cavité dans la portion de gros diamètre et la cavité dans la portion de petit diamètre sont interreliées.

3. Nanofibre selon la revendication 1 ou 2, dans laquelle le polymère hydrosoluble est un élément ou une combinaison de deux éléments, ou plus, parmi le pullulane ou un polymère synthétique choisi dans le groupe consistant en l'alcool polyvinylique partiellement saponifié, l'alcool polyvinylique faiblement saponifié, la polyvinylpyrrolidone et l'oxyde de polyéthylène.

4. Nanofibre selon l'une quelconque des revendications 1 à 3, dans lequelle le composant huileux comprend l'extrait de camomille, le cétyl-1,3-diméthylbutyléther ou l'huile siliconée.

5. Nanofibre selon l'une quelconque des revendications 1 à 4, dans laquelle le composant huileux comprend un premier composant huileux et un second composant huileux,
la cavité comprend une pluralité de cavités discrètement formées sur toute la longueur de la nanofibre, et
les cavités contiennent à la fois le premier composant huileux et le second composant huileux.

6. Nanofibre selon l'une quelconque des revendications 1 à 5, contenant un agent fonctionnel volatil ayant une pression de vapeur de 13,3 Pa ou moins à 20 °C.

7. Feuille de nanofibres comprenant la nanofibre selon l'une quelconque des revendications 1 à 6.

8. Feuille de nanofibres selon la revendication 7, ayant une surface qui doit être fixée à la peau humaine, ou à la peau ou aux dents de mammifères autres que l'être humain.

9. Feuille de nanofibres selon la revendication 8, qui est destinée à une utilisation comme feuille devant être fixée à la peau humaine, et qui a une épaisseur de 50 nm à 1 mm.

10. Utilisation de la feuille de nanofibres selon l'une quelconque des revendications 7 à 9, comme feuille hydratante, feuille cosmétique, ou feuille médicale.

11. Méthode pour la production de la nanofibre selon la revendication 1, comprenant l'électrofilage d'une émulsion H/E ayant un polymère hydrosoluble dissous dans une phase aqueuse et un composant huileux contenu dans une phase huileuse.

12. Méthode pour la production de la nanofibre selon la revendication 11, dans laquelle l'émulsion H/E est préparée en mélangeant un premier liquide qui est une solution aqueuse d'un polymère hydrosoluble dissous dans l'eau et un second liquide qui est une émulsion H/E ayant un composant huileux contenu dans une phase aqueuse.

13. Méthode pour la production de la nanofibre selon la revendication 12, dans laquelle l'émulsion H/E du second liquide est préparée en utilisant un tensioactif non ionique comme émulsifiant.

14. Méthode pour maintenir une surface d'un objet hydraté en mouillant la surface de l'objet avec de l'eau ou un liquide aqueux contenant de l'eau avant d'appliquer la feuille de nanofibres selon les revendications 7 à 9 sur la surface de l'objet, ce qui permet de détruire la cavité de la nanofibre de sorte que le composant huileux retenu dans la cavité s'écoule et que le composant huileux s'écoulant recouvre la surface de l'objet, l'objet étant la peau humaine.
